# EUROPEAN PATENT APPLICATION

(11) **EP 4 101 821 A2**
(43) Date of publication of application: **14.12.2022**
(21) Application number: 22174025.1
(22) Date of filing: 18.05.2022
(51) Int. Cl.: C03C 17/00, A61J 1/14, B65B 21/02, B65D 23/08, C03C 17/32

(54) **PROCESSING METHOD FOR PACKAGING UNITS**

(30) Priority: 18.05.2021 US 202163190095 P
(71) Applicant: Gerresheimer Glas GmbH, 40468 Düsseldorf (DE)
(72) Inventor: ROSENMAN, Scott, Mount Laurel, NJ 08054 (US); MILLER, Braden, Havertown, PA 19083 (US); HAYES, Robert, Youngsville, NC 27596 (US); HAAF, Lothar, 97255 Gelchsheim (DE); FITZPATRICK, Sean, Turnersville, NJ 08012 (US); CUEVAS, Ramses, 76905 Corregidora, Queretaro (MX); FRAAS, Andreas, 92224 Amberg (DE); GSCHWENDTNER, Lukas, 93055 Regensburg (DE)
(74) Representative: Fish & Richardson P.C.

(57) **Abstract**

The present disclosure describes a method that includes receiving a plurality of packaging units made of glass; coating at least a portion of an outer surface of each packaging unit with low-friction coating; curing the low-friction coating applied to the outer surface of each packaging unit; receiving a container configured to store the plurality of packaging units; and arranging each packaging unit in the container with the outer surface of each packaging unit able to touch the outer surface of one or more adjacent packaging units.

## Description

This disclosure relates to a method of processing packaging units and a combination that includes a container and a plurality of packaging units.

In industrial contexts, products are generally transported and sold in packaging units. Packaging units can include vials, cartridges, ampoules, bottles, or pre-fillable syringes. In many industries, these different types of packaging units are collectively known as "primary packaging," i.e., the packaging that comes into direct contact with an end product. The end product may be a food product, a cosmetic product, or a pharmaceutical product, for example. Primary packaging can undergo numerous manufacturing processes before being filled with the end product. During these processes, primary packaging is often processed in batches.

The present disclosure aims to provide a method for processing packaging units made of glass.

According to a first general aspect of the present disclosure, a method includes receiving a plurality of packaging units made of glass, coating at least a portion of an outer surface of each packaging unit with low-friction coating, curing the low-friction coating applied to the outer surface of each packaging unit, receiving a container configured to store the plurality of packaging units, and arranging each packaging unit in the container with the outer surface of each packaging unit able to touch the outer surface of one or more adjacent packaging units.

The packaging units may be vials, ampoules, cartridges, or syringe bodies and, thus, primary packaging in the food, cosmetic, or pharmaceutical industries, for example.

The low-friction coating may include fluoropolymer coating. For example, the low-friction coating may include perfluoroalkoxy alkane (PFA) coating.

Coating at least a portion of an outer surface of each packaging unit with low-friction coating may include sealing an opening of the packaging unit, and rotating the packaging unit as the low-friction coating is sprayed onto at least a portion of an outer surface of the packaging unit. Optionally, a pressure differential may be created to attract the sprayed coating to the outer surface of the packaging unit. Alternatively, coating at least a portion of an outer surface of each packaging unit with low-friction coating may include submersing the outer surface of each packaging unit in the low-friction coating. For example, the packaging units may be submerged to a certain point (e.g., the neck of a vial) to prevent the low-friction coating from entering the packaging unit through the opening.

Once the plurality of packaging units is arranged in the container, the container may be closed for further transport. For example, the container may be sealed with a flexible film lid or closed with a rigid or semi-rigid lid. In some instances, the closed container may be arranged in a sterile bag that is permeable to vapors but not to liquid, for example.

The plurality of packaging units may be sterilized. For example, the method may include sterilizing the plurality of packaging units before or after the packaging units have been arranged in the container.

According to a second general aspect of the present disclosure, a combination includes a container, and a plurality of packaging units arranged in the container such that an outer surface of each packaging unit is able to touch the outer surface of one or more adjacent packaging units, wherein the packaging units are made of glass, and the outer surface of each packaging unit comprises a low-friction coating.

The low-friction coating may include fluoropolymer coating, in particular, perfluoroalkoxy alkane (PFA) coating.

The combination may further include a flexible lid that seals the container or a rigid or semi-rigid lid that closes the container.

These and other embodiments described herein may provide one or more of the following benefits. The low-friction coating may suppress mechanical damage to packaging units and provide a more robust packaging unit. Increased robustness may reduce the number of rejected units and increase handling speed across various manufacturing processes. The batches of packaging units may be densely packed in transport containers, further improving the efficiency of downstream processes.

Certain embodiments will now be described, by way of example only, with reference to the accompanying drawings, in which:
Figure 1 shows a schematic overview of a method according to the present disclosure;
Figure 2 is a schematic side view of a vial that can be used in the method according to the present disclosure;
Figures 3A and 3B each show a schematic overview of an arrangement for coating the outer surface of a packaging unit;
Figure 4 is a schematic top view of a combination of a container and plurality of packaging units according to the present disclosure; and
Figure 5 shows a schematic cross-sectional view of a further combination according to the present disclosure.

Like reference numbers and designations in the various drawings indicate like elements.

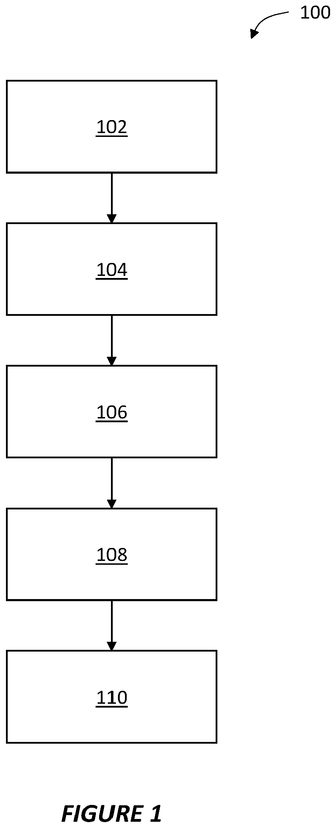

Figure 1 shows a schematic overview of a method 100 according to the present disclosure. The method 100 includes receiving 102 a plurality of packaging units made of glass, coating 104 at least a portion of an outer surface of each packaging unit with low-friction coating, curing 106 the low-friction coating applied to the outer surface of each packaging unit, receiving 108 a container configured to store the plurality of packaging units, and arranging 110 each packaging unit in the container with the outer surface of each packaging unit able to touch the outer surface of one or more adjacent packaging units.

In the following description, vials are described as an example of packaging units. Vials can be used to store pre-portioned amounts of a liquid or non-liquid end product and often serve as primary packaging, e.g., in the pharmaceutical industry. However, the method 100 is not restricted to vials. Other types of packaging units can include ampoules, cartridges, or syringe bodies to name a few examples.

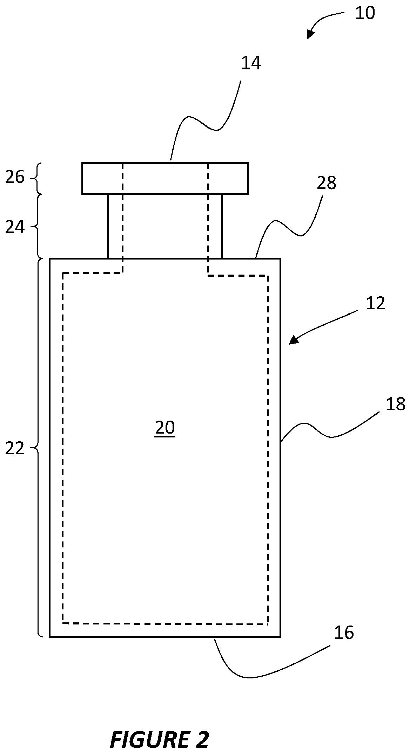

Figure 2 is a schematic side view of a vial 10. The vial 10 includes a substantially cylindrical body 12 made of glass that includes an opening 14, a bottom surface 16, and an outer surface 18. The body 12 defines an inner space 20 that can be filled with a liquid or non-liquid product. The inner space 20 is communicated with the opening 14. The body 12 comprises a large diameter section 22, a neck section 24 that connects to the large diameter section 22, and an optional opening rim 26. A shoulder or transition region 28 is formed between the large diameter section 22 and the neck section 24. Although Figure 2 shows the body 12 with sharp corners, the body 12 may include curved surfaces, particularly in the neck section 24, the transition region 28, and the bottom surface 16.

As described in reference to Figure 1, the method 100 can include receiving a plurality of the vials 10 shown in Figure 2. For example, the vial 10 can be manufactured by forming and annealing a glass material, such as borosilicate glass.

The method 100 can further include coating at least a portion of the vial's outer surface 18 with low-friction coating. In some instances, the entire outer surface 18 of the vial body 12 can be coated. However, the low-friction coating can also be limited to a portion of the outer surface 18 that corresponds to the large diameter section 22, for example. This part of the outer surface 18 is likely to come into contact with other vials (so-called glass-on-glass contact) that can lead to mechanical damage, such as scratches and abrasions. The transition region 28, the neck section 24, and the optional opening rim 26 may remain uncoated to prevent the coating from entering the inner space 20 via the opening 14.

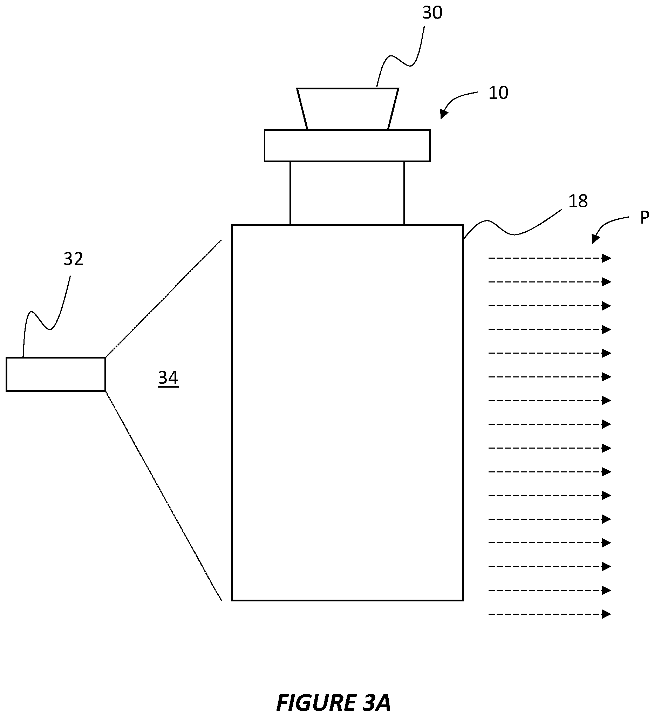

Figure 3A shows a schematic overview of an arrangement for coating at least a portion of the vial's outer surface 18 with low-friction coating. The opening 14 of the vial 10 may be sealed, as schematically shown by a stopper 30. The vial 10 may be rotated as a spray gun 32 directs a spray 34 of low-friction coating towards the outer surface 18 of the vial 10. In some instances, a pressure differential P (e.g., a vacuum) may be created on the opposite side of the vial 10 to the spray gun 32 to attract the spray 34 towards the outer surface 18 of the vial 10. As illustrated, the spray 34 is directed to the large diameter section 22 (Figure 2) of the vial 10. In some instances, the spray 34 may cover additional parts of the vial 10, such as the neck section 24.

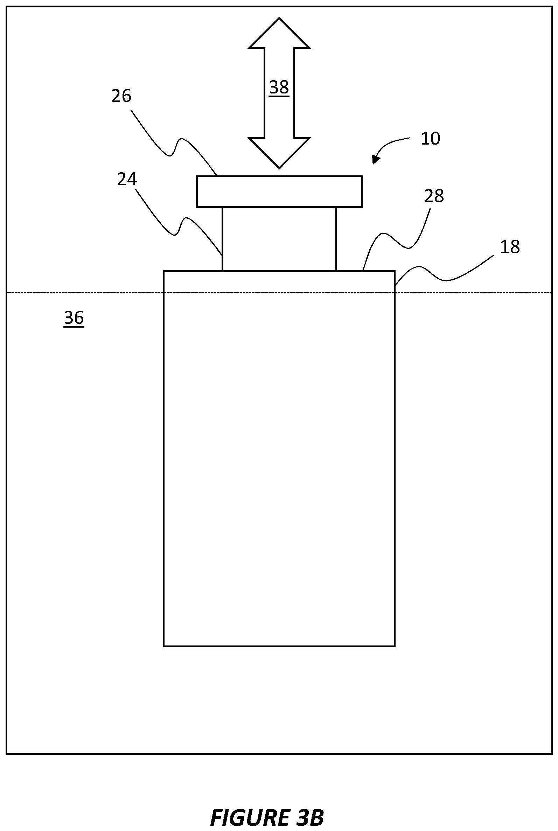

Figure 3B shows a schematic overview of a further arrangement for coating at least a portion of the vial's outer surface 18 with low-friction coating. Specifically, the vial 10 can be submersed in a bath 36 of low-friction coating, as indicated by the arrow 38. The vial 10 may be submersed so that certain portions of the body remain exposed and free from coating, e.g., the transition region 28, the neck section 24, and the opening rim 26.

Although Figures 3A and 3B each show an individual vial 10, the arrangements can be used to coat multiple vials 10 at once. Further, although the arrangements may be used with various types of low-friction coating, the spray 34 and the bath 36 may include fluoropolymer coating in some examples. More specifically, the spray 34 and the bath 36 may include perfluoroalkoxy alkane (PFA) coating.

Following the coating, the vials 10 are cured to allow the low-friction coating to adhere to the outer surface 18. The curing process may evaporate or volatize processing aids that are incorporated into the spray 34 or the bath 36. In some instances, the vials 10 may be washed after curing to remove any remaining particles of the low-friction coating that have not adhered to the vial 10. In this way, a low-friction and scratch-resistant coating is formed on the vials 10. The reduced coefficient of friction may suppress mechanical damage to the vials 10. The reduced likelihood of damage can also increase the speed and efficiency of handling in subsequent processes, as described below in more detail.

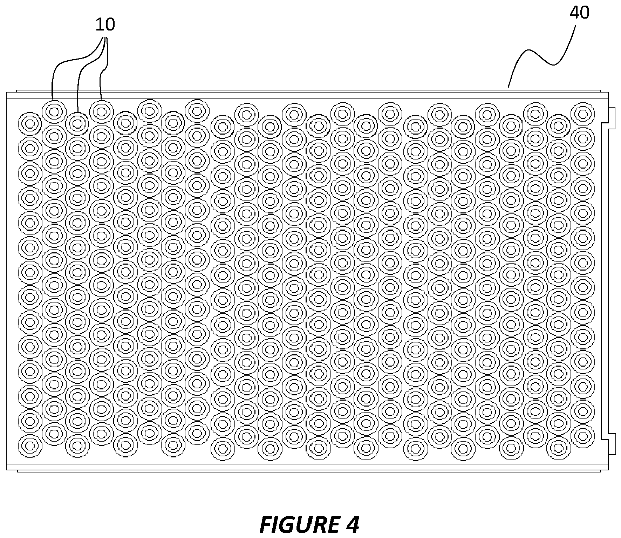

Figure 4 is a schematic top view of a container 40 with a plurality of vials 10 arranged inside. Before being placed in the container 40, the vials 10 have been provided with a low-friction coating as previously described. As shown in Figure 4, the vials 10 are arranged in a plurality of rows such that an outer surface of each vial 10 is able to touch the outer surface of one or more adjacent vials 10. In this instance, "able to touch" can mean that the vials 10 are placed adjacent to one another without an intermediate support structure between the vials 10. The low-friction coating enables the vials 10 to be packaged independently of an intermediate support structure (commonly referred to as a "nest"). Accordingly, the vials 10 can be packed with higher density than may otherwise be the case. Further, the vials 10 can be packed in different arrangements of rows in the same container 40.

In addition to allowing tight packing of the vials 10 inside of the container 40, the low-friction coating may provide a more homogenous thermal distribution across the glass surface of the vial 1 0. This homogenous thermal distribution may be useful during a lyophilization procedure.

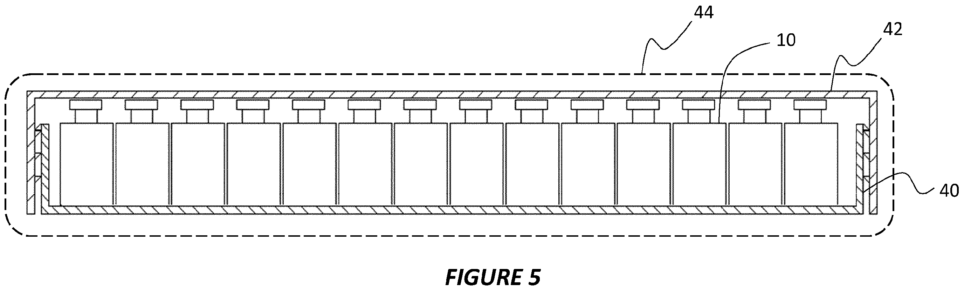

After the vials 10 have been arranged in the container 40, the container 40 may be closed for further transport and processing of the vials 10. Figure 5 shows a schematic cross-sectional view of a plurality of vials 10 stored in a container with a lid 42. The lid 42 can be formed of a rigid or semi-rigid material, such as plastic. The lid 42 may releasably latch to the container 40. As illustrated, the closed container 40 is arranged in an optional sterile bag 44 that is permeable to vapor but not to liquid. For example, the bag 44 may be made from a gas-permeable foil, such as Tyvek^{®}.

Although the container 40 of Figure 5 is closed by a rigid or semi-rigid lid 42, other implementations of the method may use a different type of container 40, colloquially referred to as a "tub." The tub may have an opening that is sealed by a flexible film lid, e.g., a gas-permeable foil made of Tyvek.

The plurality of vials 10 may be sterilized. In some instances, the vials 10 may be sterilized while inside the container 40, as shown in Figure 5. In other instances, the vials 10 may be sterilized prior to their arrangement in the container 40. When heat is used during the sterilization process, sterilization temperatures may be maintained below the curing temperatures, e.g., 320 to 400°C. In some instances, the sterilization temperatures may be maintained as low as at or below 280 °C.

A number of embodiments have been described. Nevertheless, numerous alternative embodiments within the scope of the claims will be readily appreciated by those skilled in the art. The presently described embodiments are not to be taken as limiting the scope of the invention.

## Claims

1. A method comprising:
receiving a plurality of packaging units made of glass;
coating at least a portion of an outer surface of each packaging unit with low-friction coating;
curing the low-friction coating applied to the outer surface of each packaging unit;
receiving a container configured to store the plurality of packaging units; and
arranging each packaging unit in the container with the outer surface of each packaging unit able to touch the outer surface of one or more adjacent packaging units.

2. The method according to claim 1, wherein the packaging units are vials, ampoules, cartridges, or syringe bodies.

3. The method according to claim 1 or 2, wherein the low-friction coating comprises fluoropolymer coating.

4. The method according to claim 3, wherein the low-friction coating comprises perfluoroalkoxy alkane (PFA) coating.

5. The method according to any one of the preceding claims, wherein coating at least a portion of an outer surface of each packaging unit with low-friction coating comprises:
sealing an opening of the packaging unit; and
rotating the packaging unit as the low-friction coating is sprayed onto at least a portion of an outer surface of the packaging unit.

6. The method according to claim 5, further comprising creating a pressure differential to attract the sprayed coating to the outer surface of the packaging unit.

7. The method according to any one of claims 1 to 4, wherein coating at least a portion of an outer surface of each packaging unit with low-friction coating comprises submersing the outer surface of each packaging unit in the low-friction coating.

8. The method according to any one of the preceding claims, further comprising sealing the container with a flexible film lid.

9. The method according to any one of the preceding claims, further comprising closing the container with a rigid or semi-rigid lid.

10. The method according to claim 8 or 9, further comprising arranging the container in a sterile bag.

11. The method according to any one of the preceding claims, further comprising sterilizing the plurality of packaging units after the packaging units have been arranged in the container.

12. The method according to any one of claims 1 to 10, further comprising sterilizing the plurality of packaging units before the packaging units are arranged in the container.

13. A combination comprising:
a container; and
a plurality of packaging units arranged in the container such than an outer surface of each packaging unit is able to touch the outer surface of one or more adjacent packaging units, wherein the packaging units are made of glass, and the outer surface of each packaging unit comprises a low-friction coating.

14. The combination of claim 13, wherein the low-friction coating comprises fluoropolymer coating, in particular, perfluoroalkoxy alkane (PFA) coating.

15. The combination of claim 13 or 14, further comprising a flexible lid that seals the container or a rigid or semi-rigid lid that closes the container.
